# EUROPEAN PATENT APPLICATION

(11) **EP 0 635 243 A2**
(43) Date of publication of application: **25.01.1995**
(21) Application number: 93630086.2
(22) Date of filing: 11.11.1993
(51) Int. Cl.: A61B 17/28

(54) **Forceps**

(30) Priority: 21.07.1993 US 94627
(71) Applicant: Lolagne, Fritz, Petit-Goave (HT)
(72) Inventor: Lolagne, Fritz, Petit-Goave (HT)
(74) Representative: Schmitz, Jean-Marie

(57) **Abstract**

A forceps structure including a pair of crossed arms (10,12), a stud rotationally connecting the arms and a grasping opening (10a,12a) defined by the ends of the arms (10,12,) which conforms closely to the cross-sectional shape of the vas deferens or the vagus nerve of the human body, for use in surgical procedures.

## Description

### Field of the Invention

This invention relates primarily to a forceps structure, and more particularly to a forceps structure useful in vasectomies, surgical interventions in the lower esophagus or stomach and other surgical procedures.

### Background of the Invention

With over-population in the world today, family planning, sterilization and particularly the surgical procedure of vasectomy have become common varieties of treatment for the purpose of reducing world population. Of course, the foregoing statement is applicable as well to pockets of over-population and treatment therefor which extends to various locations throughout the world.

Considering vasectomy, in particular, a commonly used instrument in the procedure is a standard forceps design, such as the Allis forceps. Such standard forceps are used to elevate a duct in the scrotum used for carrying semen in a male. The duct is commonly referred to as the vas deferens, and the purpose of its elevation is to expose such duct in order to enable the procedure for cutting it, so as to, at least temporarily, interrupt the flow of semen.

A very important consideration in the surgical procedure is the time necessary for the operation. There are two reasons for this consideration: firstly, the timing of any surgery is critical and; secondly, the great number of procedures done in this area unnecessarily burdens the surgeon if the procedure takes longer than it should. Furthermore, for the purposes of accuracy during the surgery, and in order to reduce the time for the procedure, it is important that the forceps hold the vas deferens during the procedure without slippage thereof.

It is also a consideration that the incision for the proceudre be as small as possible.

With standard forceps used for vasectomies, the scissors-like construction includes, of course, a locking mechanism for the two arms and an opening defined by the ends of such arms opposite the ends which have the grasping handles. Such openings, defined at the ends of the arms, are typically elongated openings, so that the grasping action is accomplished with a closed, but elongated loop. This allows slippage, which in turn affects the timing of the procedure, and also requires a larger incision than that necessary with the present invention.

The forceps art, as it exists at this time, can be represented by three patents, British patent No. 4688, published in 1894; U. S. Patent No. 2,642,871 to Thuerig; and U. S. Patent No. 2,397,823 to Walter. None anticipate the invention, which is the subject of this application, generally because of the shape of the grasping openings. The British patent to Arnold has a more eliptical configuration, which is more suitable for grasping a bullet than it is for grasping the vas deferens, or even the vagus nerve during intervention in the lower esophagus or stomach. Also, Theurig presents an unsuitable shape as does the Walter patent. More particularly, Theurig is for grasping sponges and Walter is for holding needles or sutures.

### Objects and Summary of the Invention

Accordingly, a primary object of the present invention is to provide a forceps with a grasping opening defined in a shape smaller than those commonly used, and conforming closely to the shape of the vas deferens or the vagus nerve.

A further and more particular object is to provide a forceps having a grasping opening which is not unduly elongated in order to prevent slippage particularly during the performance of a vasectomy.

A still further object of the present invention is to provide a forceps structure with a corresponding opening defined in a shape which does not require a large incision for surgical procedures in order to insert the instrument.

These and other objects of the present invention are featured in a forceps structure which includes a pair of arms, defining at their ends, opposite the handle ends, openings which conform closely with the cross-sectional shape of the vas deferens or the vagus nerve.

### Brief Description of the Drawings

Other objects, features and advantages of the present invention will become apparent by reference to the following more detailed description of a preferred, but nonetheless, illustrative embodiment, with reference to the accompanying drawings, wherein:
FIG. 1 is a plan view of the forceps of the present invention, showing particularly the small but suitable openings defined by the arms thereof, and showing the forceps in pre-grasping or open position;
FIG. 2 is a view similar to that of FIG. 1, but showing the invention forceps in closed or grasping position;
FIG. 3 is a view of the ends of the arms of the invention forceps, in a closed or grasping position, with respect to a vas deferens or nerve, and particularly showing the suitability of the opening defined by the ends of the arms with respect to the grapsed vas or nerve; and
FIG. 4 is a side view of the forceps grasping end of FIG. 3.

### Detailed Description of the Preferred Embodiment

Referring to the drawings, as with all forceps, the instrument of the present invention includes arms 10, 12, with each having a handle 14, 16, respectively. When in the grasping position, the instrument of the present invention is locked by means of sawtooth extensions 18, 20, respectively extending toward each other and inwardly from arms 10, 12.

The grasping end of the forceps, generally designated 22, is wherein the present invention differs from the prior art. More specifically, the grasping end of the forceps 22 includes a crossover stud 24, at which point arms 10, 12 intersect each other, as shown. Each arm, 10, 12 terminates in a grasping tip 10', 12', with each such tip defining a grasping opening 10a, 12a.

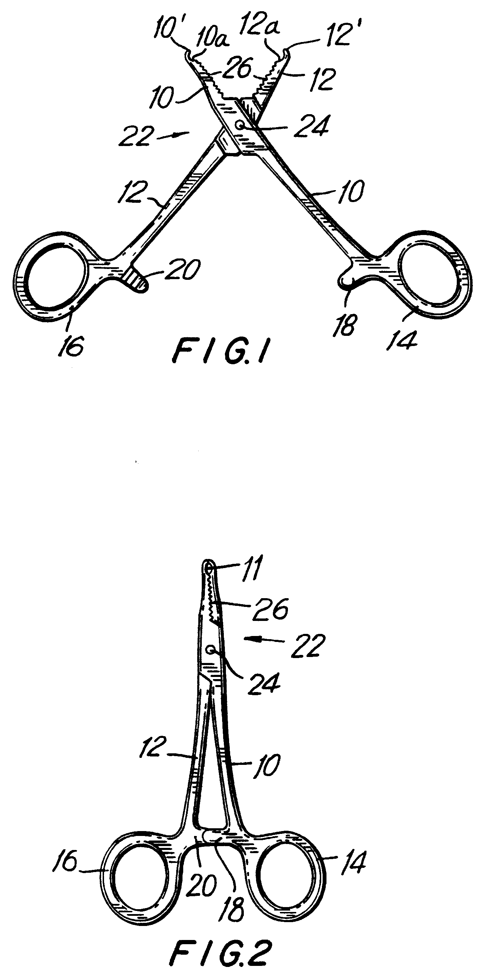

As may be seen, fron FIG. 2 particularly, the mating of such openings 10a, 12a, forms a generally circular opening, slightly elongated in the direction of the lengthwise extension of the instrument. In other words, loop 11 at the grasping end 22 of the forceps is slightly oval in shape. Sample dimensions for the opening are 0.25 cm. in width and 0.40 cm. in length. Such forceps are also facilitated in its grasping operation by means of mating serrations 26.

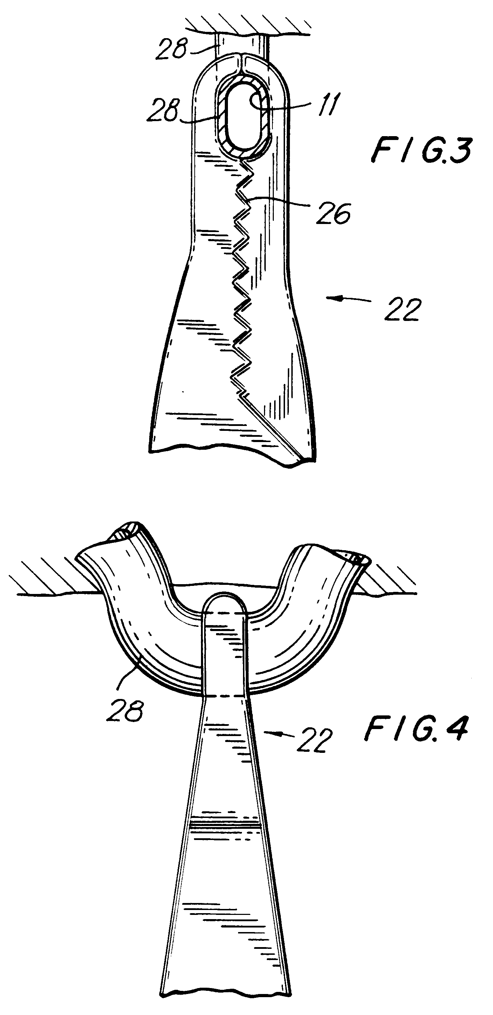

Particularly in FIGS. 3 and 4, the purpose of the forceps, which is the subject of this invention, is shown most clearly. Such purpose is a grasping operation.

In order to provide a complete understanding of the present invention, a series of use and operational steps will now be provided, with reference to FIGS. 3 and 4.

The surgeon (not shown) first makes an incision in the scrotum, assuming the purpose of the surgery is a vasectomy. Because of the compact and efficient shape of the present invention, such incision can be smaller than those provided using forceps of the prior art.

The grasping end 22 of the forceps instrument is lowered into the incision and loop 22 is caused to encircle the vas deferens 28. The instrument is then closed with serrations 26 in mating position as shown in FIG. 3, and it can be seen from FIG. 3 that, almost exactly the vas deferens 28 fills the entire loop 11 defined at the grasping end 22 of the forceps.

As shown in FIG. 4, the vas deferens 28 is then lifted by the forceps grasping end 22, and the usual cutting procedure follows.

The foregoing description of the operation of the forceps of the present invention is likewise applicable to a surgical procedure commonly performed in connection with the lifting of the vagus nerve in the lower esophagus or in the stomach. Such an operation is performed most efficiently by adding approximately six centimeters to the length of the handle of the forceps, in order to accommodate the depth of insertion, when the lower esophagus or stomach is the target area for the surgery.

All of the foregoing description is not to be considered as limiting the present invention, whose metes and bounds are to be delineated only by the following claims:

## Claims

1. A forceps for use in grasping and pulling an elongated human body part, comprising a pair of intersecting, elongated forceps arms, a stud at said intersection, a handle at a first end of each of said arms, and said arms defining a pair of facing openings for said grasping at a second end of said arms, said facing openings defining a closed, generally circularly shaped opening, when said forceps are closed, of a size approximating the cross-sectional size of said part.

2. The invention according to claim 1 wherein said size is approximately 0.40 cm. long and 0.25 cm. wide.

3. The invention according to claim 1 wherein said part is the vas deferens.

4. The invention according to claim 1 wherein said part is the vagus nerve.

5. The invention according to claim 1 wherein said part is the vagus nerve and said arms are approximately 6 cm. longer, between said stud and handles, than when said part is a vas deferens.

6. The invention according to claim 1 wherein said definition is of an oval.

7. The invention according to claim 1 wherein said definition is of an ellipse.
